# EUROPEAN PATENT APPLICATION

(11) **EP 3 875 108 A1**
(43) Date of publication of application: **08.09.2021**
(21) Application number: 19878869.7
(22) Date of filing: 29.03.2019
(51) Int. Cl.: A61K 38/48, A61P 29/00

(54) **NEW USE OF ANTIPLATELET THROMBOLYSIN**

(30) Priority: 29.10.2018 CN 201811267026
(71) Applicant: Zhaoke Pharmaceutical (Hefei) Company Limited, Anhui 230088 (CN)
(72) Inventor: CHO, Jaehyung, Hefei, Anhui 230088 (CN); LI, Xiaoyi, Hefei, Anhui 230088 (CN); DAI, Xiangrong, Hefei, Anhui 230088 (CN)
(74) Representative: Isern Patentes y Marcas S.L.
(86) International application number: PCT/CN2019/080446
(87) International publication number: WO 2020/087855

(57) **Abstract**

Provided is a use of antiplatelet thrombolysin in preparation of medications for inhibiting inflammation.

Sequence Listing

<110>  ZHAOKE PHARMACEUTICAL (HEFEI) COMPANY LIMTED
<120>  new use of antiplatelet thrombolysin
<160>  2
<170>  PatentIn version 3.3

<210>  1
<211>  130
<212>  PRT
<213>  artificial sequence
<400>  1
Asp Val Asp Cys Leu Pro Gly Trp Ser Ala Tyr Asp Gln Ser Cys Tyr
1               5                   10                  15

Arg Val Phe Lys Leu Leu Lys Thr Trp Asp Asp Ala Glu Lys Phe Cys
            20                  25                  30

Thr Glu Arg Pro Lys Gly Gly His Leu Val Ser Ile Glu Ser Ala Gly
        35                  40                  45

Glu Arg Asp Phe Val Ala Gln Leu Val Ser Glu Asn Lys Gln Thr Asp
    50                  55                  60

Asn Val Trp Leu Gly Leu Lys Ile Gln Ser Lys Gly Gln Gln Cys Ser
65                  70                  75                  80

Thr Glu Trp Thr Asp Gly Ser Ser Val Ser Tyr Glu Asn Phe Ser Glu
                85                  90                  95
Tyr Gln Ser Lys Lys Cys Phe Val Glu Lys Asn Thr Gly Phe Arg Thr
            100                 105                 110

Trp Leu Asn Leu Asn Cys Gly Ser Glu Tyr Ala Phe Val Cys Lys Ser
        115                 120                 125

Pro Pro
    130

<210>  2
<211>  125
<212>  PRT
<213>  artificial sequence
<400>  2
Gly Phe Cys Cys Pro Leu Arg Trp Ser Ser Tyr Glu Gly His Cys Tyr
1               5                   10                  15

Leu Val Val Lys Glu Lys Lys Thr Trp Asp Asp Ala Glu Lys Phe Cys
            20                  25                  30

Thr Glu Gln Arg Lys Gly Gly His Leu Val Ser Val His Ser Arg Glu
        35                  40                  45

Glu Ala Asp Phe Leu Val His Leu Ala Tyr Pro Ile Leu Asp Leu Ser
    50                  55                  60

Leu Ile Trp Met Gly Leu Ser Asn Met Trp Asn Asp Cys Lys Arg Glu
65                  70                  75                  80

Trp Ser Asp Gly Thr Lys Leu Asp Phe Lys Ala Trp Ala Lys Thr Ser
                85                  90                  95

Asp Cys Leu Ile Gly Lys Thr Asp Asp Asn Gln Trp Leu Asn Met Asp
            100                 105                 110

Cys Ser Lys Lys His Tyr Phe Val Cys Lys Phe Lys Leu
      115                 120                 12

## Description

This use claims the priority of Chinese patent use 201811267026.7, filed with the Chinese Patent Office on October 29, 2018, titled "new use of antiplatelet thrombolysin ", the entire content of which is hereby incorporated by reference.

### FIELD

The invention relates to the field of medicine, in particular to a new use of an antiplatelet thrombolysin in the preparation of a medicament for inhibiting inflammation.

### BACKGROUND

Inflammation refers to a series of complex reactions occur locally and throughout the body in response to the exogenous and endogenous injury factors that cause a variety of cell injury lesions, so as to limit and eliminate the injury factors, and to remove and absorb necrotic tissue, and repair injury. Inflammation is a defensive response of the body.

The factors that cause the body's inflammatory response include external biological factors, physical and chemical factors, autologous necrotic tissues, and allergic reactions. Vascular reaction is the central link in the inflammatory process. Some inflammatory factors can directly damage the vascular endothelium and cause increased vascular permeability. However, many inflammatory factors do not directly act on local vascular tissues, but mainly cause inflammation through the action of endogenous chemical factors. These endogenous chemical factors are chemical mediators or inflammatory mediators. When exogenous injury factors such as pathogenic microorganisms invade the body, they themselves and certain components can activate a variety of cells such as monocytes, vascular endothelial cells to release a variety of pro-inflammatory mediators such as tumor necrosis factor (TNF-α), interleukins, etc., and through exposure to activating factor XII, prekallikrein, macromolecular kininogen and the complement system, produce a series of inflammatory reactions. At the same time, invading pathogenic microorganisms and some of their components can directly or indirectly cause monocytes, macrophages and vascular endothelial cells up-regulate the expression of tissue factor, thereby initiating the coagulation process and causing thrombosis. In addition, the adhesion, activation and aggregation of platelets are the initiation and promotion factors of thrombosis, but as an inflammatory component, platelets will release a large number of cytokines after being activated, which will further enhance the inflammatory response and platelet aggregation; thrombin in the coagulation process can also induce thrombosis through its effect on white blood cells and endothelial cells, and at the same time promote the accumulation of non-cellular components of the vascular wall, the synthesis and release of inflammatory response factors by the vascular intima, and regulate the inflammatory response of plasma albumin and vascular wall cells.

Studies have shown that vascular response is a central link in the inflammatory process. After tissue injury in the acute inflammation process, hemodynamic changes will soon occur, including changes in blood flow and vascular caliber, such as short-term constriction of arterioles, vasodilatation, acceleration of blood flow, slowing of blood flow, and stagnation of blood flow and so on, and the speed of its occurrence depends on the severity of the injury. In this process, under the action of inflammatory mediators, the permeability of the blood vessel wall increases, and plasma proteins and some liquids leak out from the venous ends of capillaries and venules, causing hemoconcentration and increased blood viscosity, leading to slow blood flow, thrombosis formed. At the same time, the hydrostatic pressure in the capillaries and venules increases, resulting in a large amount of plasma exuding, resulting in tissue edema. As the blood flow slows down, red blood cells aggregate into clusters, white blood cells escape, resulting in thrombosis and accelerated inflammation.

Normal vascular endothelial cells are smooth and complete, which is not conducive to platelet adhesion. However, after being activated or injured, the natural barrier function is destroyed, and the injured endothelial cells can release tissue factor and thrombomodulin to activate the coagulation system. A large number of procoagulant factors and substances produced by it can promote vasoconstriction and decrease the function of fibrinolysis. In addition, the slow blood flow is prone to stagnation and vortex in the depression of the venous valve, aggravating the damage of the blood vessel wall, and on the surface of the damaged vascular endothelial cell the expression of a large number of adhesion molecules causes monocytes, neutrophils, and platelets to roll, adhere and aggregate on their surfaces, leading to inflammatory reactions. At the site of ischemic vascular injury, the exposed fibrous collagen binds to GPVI, induces platelet activation and the functional up-regulation of GPIIb/IIIa receptors, and the activated platelets release polyphosphate by themselves. The polyphosphate and the negatively charged surface act and activate factor XII, the endogenous coagulation pathway initiating enzyme. In addition to triggering thrombus formation through fibrin production, FXII also promotes the activation of the contact kinin system: FXIIa cleaves plasma kallikrein to form the active serine protease plasma kallikrein, thereby cleaving high-molecular kallikrein to release inflammatory peptides Kinins BK, and BK bind to endothelial cell receptors to initiate a signal cascade, induce endothelial cell damage and cause angioedema, pro-inflammatory cytokine expression, and induce glial cell activation and inflammation. At the same time, ischemic injury activates circulating white blood cells such as T cells and neutrophils, leading to aseptic inflammation, including the up-regulation of chemical inducers, chemokines, and adhesion molecules of endothelial cells and immune cells. T lymphocytes are recruited through P-selectin/P-selectin glycoprotein ligand-1 (PSGL-1) and through intercellular adhesion molecule (ICAM-1)/lymphocyte function-associated antigen-1[LFA-1] and vascular cell adhesion molecule-1 (VCAM-1)/Very late antigen-4 (VLA-4) binds stably to blood vessels, and interacts with activated platelets through CD40/CD40L to form a stable thrombus. Neutrophils interact with platelets through macrophage-1 antigen [MAC-1]/GP I ba and P-selectin/PSGL-1, and participate in fibrin cross-linking through MAC-1/fibrin interactions, and induce foreign The source tissue factor TF/FVIIa pathway to trigger the activation of thrombin, release inflammatory response factors, and regulate the inflammatory response of plasma leukocytes and vascular wall cells.

Under normal circumstances, the inflammatory response plays a protective role in the body, but when the inflammatory response is too strong, the production of a large number of inflammatory mediators will cause the body's anti-inflammatory-pro-inflammatory balance imbalance, pro-inflammatory mediators such as TNF-α, IL-1β and IL-6 can induce the production of a large number of secondary inflammatory mediators in the body, such as PGE2, PAF, and some adhesion factors. These inflammatory mediators can mediate the interaction between inflammatory cells and vascular endothelial cells, reduce the body's anti-inflammation and cause metabolic dysfunction , causing systemic inflammatory response syndrome.

### SUMMARY

The inventor unexpectedly discovered that antiplatelet thrombolysin can reduce platelet-neutrophil aggregation and inhibit inflammation.

The α chain amino acid sequence of antiplatelet thrombolysin is shown in SEQ ID NO. 1, and the β chain amino acid sequence is shown in SEQ ID NO.2; or the antiplatelet thrombolysin is derived from its α chain amino acid sequence by substitution, deletion, or addition of one or more amino acids and has at least 95% identity with SEQ ID NO. 1, or is derived from its β chain amino acid sequence by substitution, deletion, or addition of one or more amino acids and has at least 95% identity with SEQ ID NO. 2, and the antiplatelet thrombolysin has an activity of inhibiting inflamation.

The antiplatelet thrombolysin (APT, anfibatide) of the present invention can inhibit the binding of activated αMβ2 integrin to platelets, increase blood flow speed, reduce platelet-neutrophil aggregation, and is used in a mouse middle cerebral artery occlusion model (MCAO) and reperfusion models. The antiplatelet thrombolysin has a significant protective effect on brain tissue damage in mice, reduces infarct volume and improves nerve defects.

Preferably, the inflammation includes acute inflammation and chronic inflammation; more preferably, the inflammation includes degenerative inflammation, exudative inflammation, proliferative inflammation and specific inflammation.

Preferably, the medicament of the present invention is a chemical pharmaceutical preparation or a biological preparation.

Preferably, the medicament comprises antiplatelet thrombolysin and pharmaceutically acceptable excipients.

Preferably, the medicament of the present invention is an oral preparation or an injection.

More preferably, the oral preparations are tablets, capsules, pills, granules, dripping pills, microcapsules or pellets.

Preferably, the medicament of the present invention is an external preparation. More preferably, the external preparation is a tincture, an ointment, a cream, a lotion, a rinse, a liniment or a gel.

Experiments have confirmed that the antiplatelet thrombolysin of the present invention can inhibit the interaction between platelets and leukocytes mediated by αMβ2 integrin. Antiplatelet thrombolysin can reduce the aggregation of neutrophils, and can also reduce the binding between platelets and neutrophils, thereby inhibiting inflammation.

The new application of antiplatelet thrombolysin provided by the present invention can supplement the deficiency of existing drugs in treating inflammation. In vivo and in vitro experiments have confirmed that antiplatelet thrombolysin can inhibit the binding of activated αMβ2 integrin to platelets, increase blood flow speed, reduce platelet-neutrophil aggregation, and in the mouse middle cerebral artery occlusion model (MCAO) and reperfusion model, antiplatelet thrombolysin has a significant protective effect on brain tissue damage in mice, reduces infarct volume and improves neurological deficits. Bederson and grip test scores show that it can inhibit inflammation and have a good clinical application prospect.

### BRIEF DESCRIPTION OF DRAWINGS

Figure 1 shows that antiplatelet thrombolysin inhibits the binding of mouse platelets to αMβ2 integrin;
Figure 2 shows that antiplatelet thrombolysin inhibits the binding of human platelets to αMβ2 integrin;
Figure 3 shows the effect of antiplatelet thrombolysin in reducing TNF-α-induced neutrophil cell aggregation in wt mice;
Figure 4 shows that antiplatelet thrombolysin reduces TNF-α-induced platelet-neutrophil binding in wt mice;
Figure 5 shows the effect of antiplatelet thrombolysin in reducing the aggregation of human neutrophils;
Figure 6 shows the effect of antiplatelet thrombolysin in reducing the binding between human platelets and neutrophils;
Figure 7 shows images of platelets and neutrophils at different time points. From left to right, they are BSA group, 25ng/g anfibatide group, and 50g/g anfibatide group. From top to bottom, they are 0s, 30s, and 60s maps, arrow indicates the direction of blood flow, green is to mark platelets, and red is to mark neutrophils;
Figure 8 shows the blood flow velocity comparison chart of the drug administration group, from left to right are the BSA group, the 25ng/g anfibatide group, and the 50g/g anfibatide group, respectively;
Figure 9 shows the platelet-neutrophil count chart, from left to right are the control IgG group, anti PDI group, BSA group, anfibatide group, IgG+BSA group, and anti PD-I+anfibatide group;
Figure 10 shows the brain tissue slices of mice with ischemia/reperfusion induced stroke. From left to right, they are sham operation group, BSA group, 5ng/ganfibatide group, and 25ng/g anfibatide group;
Figure 11 shows the experimental results of infarct volume in mice with ischemia/reperfusion induced stroke. The ordinate is the infarct volume. From left to right, they are sham operation group, BSA group, 5ng/ganfibatide group, and 25ng/g anfibatide group;
Figure 12 shows the Grip score of ischemia/reperfusion induced stroke mice. From left to right, they are sham operation group, BSA group, 5ng/g Anfibatide group, and 25ng/g Anfibatide group;
Figure 13 shows the Bederson score of ischemia/reperfusion-induced stroke mice, from left to right are sham operation group, BSA group, 5ng/g Anfibatide group, 25ng/g Anfibatide group.

### DETAILED DESCRIPTION

The present invention provides use of antiplatelet thrombolysin in the preparation of a medicament for inhibiting inflamation. Those skilled in the art can achieve it in view of this disclosure and appropriate improvement of the process parameters. In particular, it should be pointed out that all similar substitutions and modifications are obvious to those skilled in the art, and they are all deemed to be included in the present invention. The method and use of the present invention have been described through the preferred examples. It is obvious that those skilled in the art can make changes or appropriate modifications and combinations to the methods and uses described herein without departing from the content, spirit and scope of the present invention to achieve and apply the technology of the present invention.

The reagents used in the present invention are all common commercially available products, and all are available in the market.

In order to enable those skilled in the art to better understand the technical solutions of the present invention, the present invention will be further described in detail below in conjunction with specific examples.

### Example 1 Antiplatelet thrombolysin inhibits the binding effect of αMβ2 integrin and platelets

Mouse platelets and human platelets were pre-incubated at 37 °C respectively with control IgG (10 µ g/ml), BD34 (anti-PDl antibody, 10 µ g/ml), BSA (bovine serum albumin, 0.2 µg/ml) and anfibatide (antiplatelet, thrombolysin 0.2 µ g/ml) for 30 minutes, and then washed with HEPES-Tyrode buffer (20 mM HEPES, pH 7.4, 136 mM NaCl, 2.7 mM KCl, 12 mM NaHCO₃, 2 mM MgCl₂, and 5.5 mM glucose). The washed platelets were incubated with recombinant human αMβ2 integrin (10µg/ml) treated with or without 0.5mM MnCl₂ for 30min at 37°C to wash away unbound αMβ2, and the remaining platelets were incubated with 5µg/ml IgG-FITC or goat anti-human β2 antibody, and tested by flow cytometry.

α Mβ2, also called Mac-1 or CD11b/CD18, is a member of the integrin family. It is the main factor that mediates the adhesion between cells and extracellular matrix and between cells. It can recognize and bind to the corresponding ligands in the extracellular matrix. It is mainly distributed in leukocytes, and is an important type of leukocyte adhesion factor that participates in the adhesion between leukocytes and endothelial cells, mediates the function of leukocytes, and thus plays a key role in inflammation and immune response.

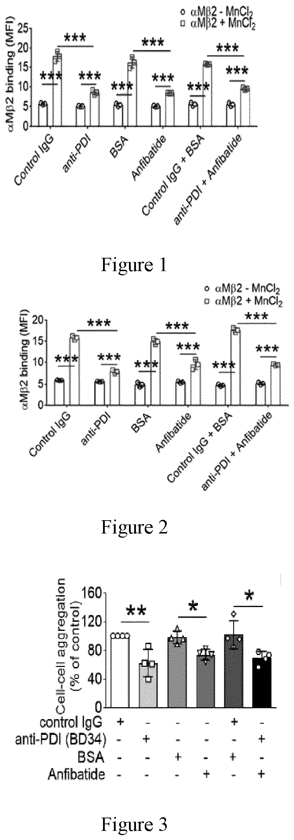

As shown in Figure 1-2, Figure 1 shows the binding effect of mouse platelets and αMβ2 integrin, and Figure 2 shows the binding effect of human platelets and αMβ2 integrin. The ordinate is the fluorescence intensity, and the abscissa corresponds to the experimental groups as follows: control IgG group, BSA group, anti PDI group, IgG+BSA group, anti PDI+anfibatide group, where anti-PDI is protein disulfide isomerase inhibitior. The bar on the left is the result of αMβ2 without MnCl₂ treatment, and the bar on the right is the result of αMβ2 treated with MnCl₂. The experimental data were statistically analyzed, n=3, *P <0.05, **P <0.01, or *** P <0.001.

The results showed that antiplatelet thrombolysin can inhibit the interaction between platelets and leukocytes mediated by αMβ2 integrin.

### Example 2 Antiplatelet thrombolysin reduces TNF-α-induced platelet-neutrophil aggregation in wt mouse model

Neutrophils are the first immune cells to reach the site of infection. They differentiate into mature granular material-carrying cells in the bone marrow and are released into the blood. In the absence of infection, the neutrophils remain in a resting state, and then exist in the peripheral blood, but once the bacteria invade, the body will quickly recruit neutrophils to the infection site, neutrophils through phagocytosis jointly produce a variety of bactericidal substances, release proteases from the particles and neutrophils, at the same time, neutrophils also secrete chemokines to recruit other immune cells to enter the infection site and jointly destroy the infectious agent.

However, over-activated neutrophils will be recruited to some important organs. In these organs, the bactericidal substances carried by activated neutrophils will be released locally, and their respective destructive effects will lead to tissue damage and further organ disorders. When neutrophils invade an organ, they will induce the accumulation of neutrophils in other important organs, leading to multiple organ failure. Inhibitors targeting different neutrophil substances can alleviate tissue damage in acute inflammation. At the same time, after activation of platelet thrombin, platelet selective receptors will be quickly expressed on the cell surface and mediate the binding and adhesion of activated platelets to neutrophils, neutrophils aggregation, and lysosomal enzyme release. Platelet-derived products can also promote the chemotaxis of neutrophils, the release of enzymes and phagocytosis, and inhibit the explosion of oxidation. On the other hand, platelets adhere to neutrophils, promote platelet aggregation, mediate vascular occlusion, and aggravate inflammation.

### experiment method:

Platelets (2x107 cells/ml) and neutrophils (1x106 cells/ml) from WT mice and PDI CKO mice (PDIflox/flox mice were hybridized with PF4-Cre mice to obtain PDI CK0 mice) were respectively labeled with DyLight488-Anti-CD42C and Alexa Fluor647-anti-Ly-6G antibody, human neutrophils and platelets were respectively labeled with Alexa Fluor 488-anti-CD41 and FITC-anti-L selectin antibodies. Neutrophils were induced with 20ng/ml TNF-a for 5 minutes, the platelets were pre-incubated with control IgG (10µg/ml), anti PDI antibody (10µg/ml), BSA (0.2µg/ml), antiplatelet thrombolysin (0.2µg/ml) and antiplatelet thrombolysin + antiPDI at room temperature for 30 minutes, then incubated with 0.025U/ml thrombin at 37°C for 5 minutes, then incubated with 50uM PPACK. The mixed platelets and neutrophils were stirred at 1000 rpm, then fixed after 5 minutes and analyzed by flow cytometry.

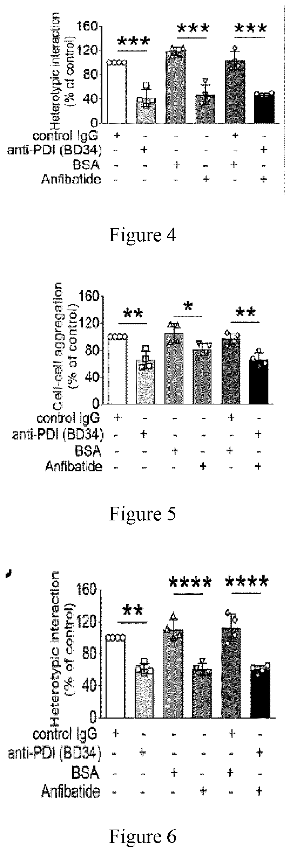

The experimental results are shown in Figure 3-6. Antiplatelet thrombolysin reduced TNF-α-induced platelet-neutrophil aggregation in the wt mouse model. The ordinate is cell-to-cell/platelet-to-neutrophil aggregation, relative to the control group. The abscissa is the experimental group, the experimental groups from left to right are: control IgG group, anti PDI group, BSA group, anfibatide group, IgG+BSA group, anti PD-I+anfibatide group. n=4, the experimental data were statistically analyzed, *P <0.05, **P <0.01, or ***P <0.001.

The test results show that antiplatelet thrombolysin can reduce the aggregation of neutrophils, and can also reduce the binding between platelets and neutrophils, thereby inhibiting inflammation.

### Example 3 Inhibition of antiplatelet thrombolysin on TNF-α-induced inflammation

TNF-α is an important pro-inflammatory cytokine, which has physiological functions such as participation in immunity, anti-tumor, anti-virus, and regulation of specific gene expression. It plays a vital role in the coordination of the body's immune-inflammation coordination signal network. If TNFa is overexpressed, it will cause inflammatory disease and natural immune disease.

WT mice were taken and injected with TNF-a into the scrotum after anesthesia. After 3 hours, they were administered with BSA 50ng/gBW and Anfibatide (25, 50ng/gBW), and recorded by real-time intravital microscope. The platelets of WT mice were labeled with calcein AM, and were respectively pre-incubated with IgG (10µg/ml), anti-PDI (10µg/ml), BSA (0.2µg/ml), anfibatide (0.2µg/ml), anfibatide+anti-PDI, 100 µl per TNF-α mouse was injected according to the number of platelets 2x10⁹/ml.

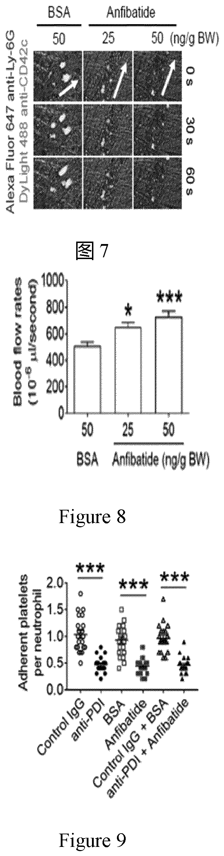

The experimental data were statistically analyzed by ANOVA and Tukey's test, *P <0.05, **P <0.01, or ***P <0.001. The test results showed that the administration of Anfibatide 25-50ng/g after TNF-α treatment had basically no effect on the rolling and adhesion of neutrophils in endothelial cells (see Figure 7). Compared with the BSA control, Anfibatide dose-dependently increased blood flow rate (see Figure 8). At the same time, the platelet transfer model was used in the intravital microscope to carry out the experiment. Treatment with 0.2µg/ml Anfibatide inhibited 50-55% of platelet-neutrophil adhesion (Figure 9), which further illustrates that antiplatelet thrombolysin reduces TNF-α-induced platelet-neutrophils aggregation in the wt mouse model.

### Example 4 Effect of antiplatelet thrombolysin on brain tissue injury in mice with ischemia/reperfusion stroke

Female and male C57BL/6 mice and WT mice (22-25g, 7-10 weeks old) were anesthetized with 2% pentobarbital and intraperitoneally injected buprenorphine (30ng/g BW) during the operation a heating blanket was used to maintain body temperature at 37°C. The left common carotid artery was exposed, the external carotid artery was dissected and the internal carotid artery was separated, the left middle cerebral artery was sealed with fibrils (diameter 0.15mm, tip diameter 0.22-0.25mm) for 1h. Laser Doppler perfusion monitoring system (PF5010, Perimed AB, Ardmore, PA) was used till the local cerebral blood flow dropped to 20% of the baseline level, which showed the embolization was successful. The fibrils were then removed and blood flow was restored to baseline. One hour after embolization, BSA or antiplatelet thrombolysin (5, 25ng/g BW, 100µl saline) was administered, buprenorphine (25ng/gBW) was injected again ip, and reperfusion was performed for 23h. Bederson and grisp were used to score animal behavior. After scoring, the mice were sacrificed and a 2mm brain tissue section was taken, stained with 2% 2,3,5-triphenyltetrazolium chloride solution at 37°C for 10 min, fixed with 4% paraformaldehyde, scanned and sliced and analyzed for infarction volume using ImageJ.

The ischemia/reperfusion brain tissue injury model is a mature inflammation model, and its inflammation is related to thrombosis. The experimental data were statistically analyzed by ANOVA and Tukey's test, n = 6-7/group, using ANOVA and Tukey test, *** P <0.001, **** P <0.0001 were significant differences.

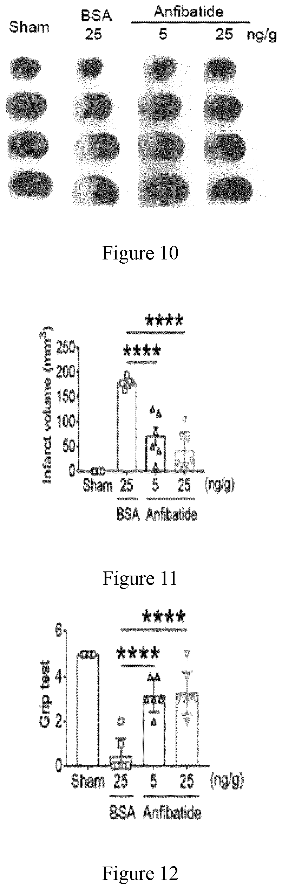

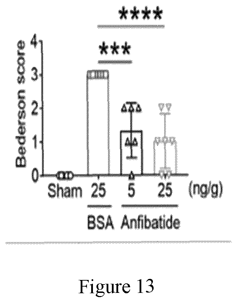

The results show that antiplatelet thrombolysin has a protective effect on brain tissue damage in mice with stroke induced by ischemia/reperfusion. Anfibatide 5-25ng/g.BW was given 1 hour after MCAO-induced local ischemia. Anfibatide at 5ng/g BW can reduce the infarct volume to 40% of the BSA control group. Compared to the BSA control, Anfibatide dose-dependently reduced infarct volume, improved nerve function, improved balance ability and exercise ability (Figure 10-13).

The above are only the preferred embodiments of the present invention. It should be pointed out that for those of ordinary skill in the art, without departing from the principle of the present invention, several improvements and modifications can be made, and these improvements and modifications should also be regarded in the protection scope of the present invention.

## Claims

1. Use of antiplatelet thrombolysin in the preparation of a medicament for inhibiting inflammation, wherein said antiplatelet thrombolysin consists of two peptide chains of α chain and β chain, and α chain amino acid sequence is shown in SEQ ID NO.1, β chain amino acid sequence is shown in SEQ ID NO. 2; or the antiplatelet thrombolysin is derived from its α chain amino acid sequence by substitution, deletion, or addition of one or more amino acids and has at least 95% identity with SEQ ID NO. 1, or is derived from its β chain amino acid sequence by substitution, deletion, or addition of one or more amino acids and has at least 95% identity with SEQ ID NO. 2, and the antiplatelet thrombolysin has an activity of inhibiting inflammation.

2. The use according to claim 1, wherein the inflammation includes acute inflammation and chronic inflammation.

3. The use according to claim 1, wherein the inflammation includes degenerative inflammation, exudative inflammation, proliferative inflammation and specific inflammation.

4. The use according to claim 1, wherein the medicament is a chemical drug or a biological preparation.

5. The use according to claim 1, wherein the medicament is an oral preparation or an injection.

6. The use according to claim 5, wherein the oral preparation is a tablet, a capsule, a pill, a granule, a dripping pill, a microcapsule or a pellet.

7. The application according to claim 1, wherein the medicament is an external preparation.

8. The application according to claim 7, wherein the external preparation is a tincture, an ointment, a cream, a lotion, a rinse, a liniment or a gel.
